# EUROPEAN PATENT APPLICATION

(11) **EP 3 679 913 A1**
(43) Date of publication of application: **15.07.2020**
(21) Application number: 19150877.9
(22) Date of filing: 09.01.2019
(51) Int. Cl.: A61H 9/00, A61M 1/00

(54) **TREATMENT HEAD FOR NEGATIVE PRESSURE TREATMENT DEVICE AND NEGATIVE PRESSURE TREATMENT DEVICE**

(71) Applicant: Lymphatouch Oy, 00510 Helsinki (FI)
(72) Inventor: KIVILAAKSO, Kristo, 02700 KAUNIAINEN (FI); LAURONEN, Juho, 00200 HELSINKI (FI)
(74) Representative: Berggren Oy, Helsinki & Oulu

(57) **Abstract**

A treatment head (100) for a negative pressure treatment device (200) and the device (200) are presented. The treatment head (100) comprises a first body part (11). The first body part (11) comprises an outer side surface (16) and an end surface (14). The first body part (11) defines a recess (10) for a filter (150) and a first opening (12) of the recess for arranging the filter (150) to the recess (10). The first opening (12) is defined in part by the end surface (14) and in part by the outer side surface (16) for facilitating a removal of the filter (150) from the recess (10).

## Description

### FIELD OF THE INVENTION

The present invention relates in general to negative pressure treatment devices. In particular, however not exclusively, the present invention concerns air flow filtering arrangements utilized in the negative pressure treatment devices.

### BACKGROUND

In known negative pressure treatment devices, such as vacuum massage devices, a filter for filtering the air flowing due to providing the negative pressure to mobilize skin tissue is arranged to the main unit of the device. The main unit is connected to a treatment head by a low-pressure hose and, thus, the filtering occurs when the air flows through the hose into the main unit. The main unit can then be opened, and the vacuum bag or filter replaced.

In the known solutions, the impurities, such as massage oil or dead skin cells, are being sucked into the low-pressure hose and then into main unit. Even by replacing the filter in the main unit, some of the impurities still remain in the treatment head and the low-pressure hose as well as part of the main unit.

### SUMMARY

An objective of the present invention is to provide a treatment head for a negative pressure treatment device and a negative pressure treatment device in order to at least alleviate some of the drawbacks in the known solutions. Another objective of the present invention is that the treatment head and the device facilitate the replacement of a filter for filtering air flow in such devices.

The objectives of the invention are reached by a treatment head and a negative pressure treatment device as defined by the respective independent claims.

According to a first aspect, a treatment head for a negative pressure treatment device is provided. The treatment head comprises a first body part. The first body part comprises an outer side surface and an end surface. The outer side surface and the end surface may, preferably, be substantially perpendicular with respect to each other. The first body part defines a recess for a filter for filtering air flow and a first opening of the recess for arranging the filter to the recess. The first opening is defined in part by the end surface and in part by the outer side surface for facilitating a removal of the filter from the recess.

In various embodiments, the treatment head may comprise a suction opening at an opposite end of the first body part with respect to a first end comprising the end surface. In addition or alternatively, the suction opening may be defined by the first body part. Still further, the suction opening may be in connection with, or even define the edge of, a negative pressure chamber arranged for pulling skin tissue into the chamber through the suction opening when the suction opening is arranged against the skin tissue.

In some embodiments, the treatment head may comprise a second opening defined by the first body part in fluid communication with the first opening. In addition, the second opening may be in fluid communication with the suction opening through a negative pressure chamber.

In various embodiments, the treatment head may comprise the second opening defined by the first body part in fluid communication with the first opening. Alternative or in addition, the filter may be arrangeable to the recess between the first and second openings for filtering air flowing between said openings. Still further, the second opening may be in fluid communication with the suction opening through a negative pressure chamber.

In various embodiments, the treatment head may comprise a second body part adapted for coupling removably with the first body part at the first end. The second body part may preferably be configured to be in fluid communication with a negative pressure source of the negative pressure treatment device, such as a vacuum pump or a fan. Furthermore, the second body part may be adapted to function as a handle for convenient moving the treatment head by an operator or user.

In various embodiments, the treatment head may comprise a sealing portion arranged to seal the coupling between the first and the second body parts. In addition, the recess may be arranged into a sealed space by the sealing portion between the first and second body parts. In some embodiments, the recess is closer to the first end than the sealing portion such that the recess is arranged to be in the sealed space between the first and second body parts.

In some embodiments, the sealing portion may comprise a groove in the first body part, wherein the groove is arranged to extent around the first body part along the outer side surface. Thus, the sealing portion may also comprise a sealing part which may, preferably, be arranged into the groove. Alternatively or in addition, the groove may be askew with respect to a plane perpendicular to a longitudinal direction of the treatment head for providing better attachment of the sealing part. The skewed groove may mean that the groove extends closer to the first end at certain portion of the outer side surface than in other portions, or that the groove runs around the first body part of the treatment head in at least two different positions, optionally continuously changing its position, with respect to the longitudinal direction of the treatment head.

In various embodiments, the treatment head may comprise a sensor configured for monitoring a presence of the filter in the recess, wherein the sensor is configured to provide an electrical signal representing the presence of the filter. The sensor may be, for example, a proximity sensor based on electromagnetic radiation, such as infrared.

According to a second aspect, a negative pressure treatment device is provided. The negative pressure treatment device comprises a treatment head according to the first aspect, or any embodiment thereof. The negative pressure treatment device further comprises a main unit comprising a controlling unit and a negative pressure source. The negative pressure treatment device further comprises a low-pressure hose or line, such as flexible or rigid hose or line, arranged for providing negative pressure produced by the negative pressure source to the treatment head.

Alternatively, the controlling unit may be arranged to the treatment head. In the various embodiments, the controlling unit may preferably comprise a processing unit, such as a processor or a microcontroller, and at least one memory element for storing program code, and, for example, measurement data of the sensor or sensors comprised in the negative treatment device.

In various embodiments of the negative pressure treatment device, the low-pressure hose or line may be attached to the treatment head for providing the negative pressure via the first and the second openings to the negative pressure chamber in connection with the suction opening.

The present invention provides a treatment head for a negative pressure treatment device, and a negative pressure treatment device. The present invention provides advantages over known solutions such that the filter can be easily replaced, for example, after using the device. This facilitates the maintenance of the device and, thus, makes it easier to maintain the hygiene of the device, such as by changing the filter between clients/patients/users.

Various other advantages will become clear to a skilled person based on the following detailed description.

The expression "a number of" may herein refer to any positive integer starting from one (1).

The expression "a plurality of" may refer to any positive integer starting from two (2), respectively.

The terms "first", "second" and "third" are herein used to distinguish one element from other element, and not to specially prioritize or order them, if not otherwise explicitly stated.

The exemplary embodiments of the present invention presented herein are not to be interpreted to pose limitations to the applicability of the appended claims. The verb "to comprise" is used herein as an open limitation that does not exclude the existence of also un-recited features. The features recited in depending claims are mutually freely combinable unless otherwise explicitly stated.

The novel features which are considered as characteristic of the present invention are set forth in particular in the appended claims. The present invention itself, however, both as to its construction and its method of operation, together with additional objectives and advantages thereof, will be best understood from the following description of specific embodiments when read in connection with the accompanying drawings.

### BRIEF DESCRIPTION OF FIGURES

Some embodiments of the invention are illustrated by way of example, and not by way of limitation, in the figures of the accompanying drawings.
Figures 1A-1D illustrate schematically a treatment head according to an embodiment of the present invention.
Figures 2A and 2B illustrate schematically treatment heads according to some embodiments of the present invention.
Figure 3 illustrates schematically a negative pressure treatment device according to an embodiment of the present invention.

### DETAILED DESCRIPTION OF SOME EMBODIMENTS

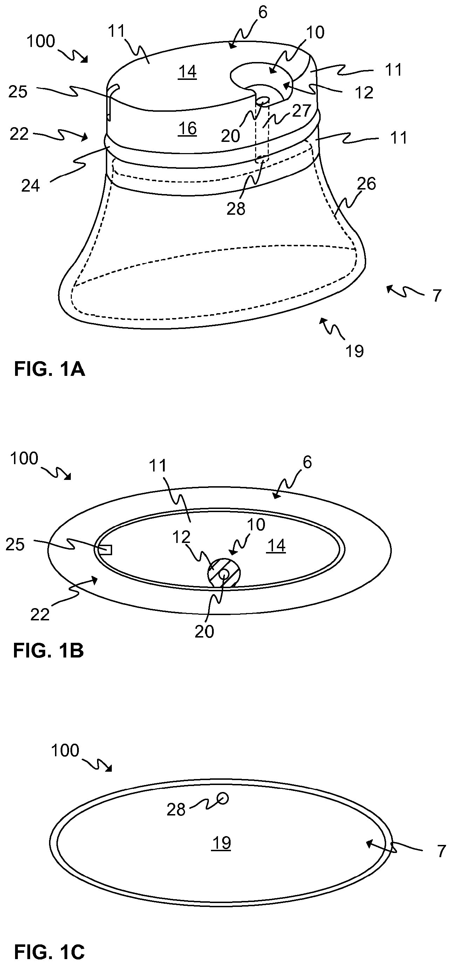

Figures 1A-1D illustrate schematically a treatment head 100 according to an embodiment of the present invention. The treatment head 100 may preferably be arranged to facilitate the replacement, and especially the removal, of a filter 150 for filtering air flow in a treatment head 100 of a negative pressure treatment device. The filter 150 may be, for example, of foam rubber or other such suitable filtering material.

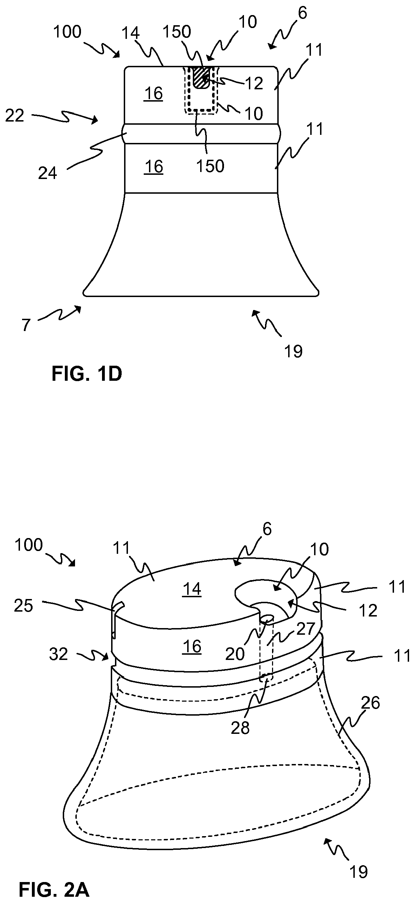

Figure 1A illustrates some features of the treatment head 100 by a perspective view, Fig. 1B from above, that is from a first side 6, Fig. 1C from below, that is from an opposite side 7 or the side of a suction opening 19, and Fig. 1D from a side.

The treatment head 100 may comprise a first body part 11 comprising an outer side surface 16 and an end surface 14. The treatment head 100 may further comprise a recess 10 for the filter 150, wherein the recess 10 may preferably be defined by the first body part 11. The treatment head 100 may comprise a first opening 12 of the recess for arranging the filter 150 to the recess 10. The first opening 12 may be arranged in or defined in part by the end surface 14 and in part to the outer side surface 16 for facilitating a removal of the filter 150 from the recess 10. The first opening 12 may be, for example, mostly (60, 70, 80, or 90 percent, for instance) at the end surface 14, that is defining a hole or cavity in the material of the first body part 11 at the end surface 14, while the rest of the first opening 12 may be at the outer side surface 16, that is defining a hole or cavity in the material of the first body part 11 at the outer side surface 16. The outer side surface 16 and the end surface 14 may, preferably, be substantially perpendicular with respect to each other.

The shape of the recess 10, and thus the filter 150, may be, for example, a cylinder or cube or other such a shape.

In some embodiments, the treatment head 100 may comprise a suction opening 19 at an opposite side 7 of the first body part 11 with respect to a first end 6 comprising the end surface 14.

According to various embodiments, the suction opening 19 may be comprised in or defined by the first body part 11, for example, at the opposite end 7 of the first body part 11 with respect to the end surface 14.

In various embodiments, the treatment head 100 may comprise a second opening 20 in the first body part 11 in fluid communication with the first opening 12.

In various embodiments, the filter 150 may be arrangeable or may be arranged to the recess 10 between the first 12 and second openings 20 for filtering air flowing between said openings 12, 20.

In various embodiments, the second opening 20 may be in fluid communication with the suction opening 19 through a negative pressure chamber 26 and, optionally, an inner fluid communication connection 27 and a third opening 28. The negative pressure chamber 26 may be arranged for pulling skin tissue into the chamber 26 through the suction opening 19 when the suction opening 19 is arranged close to or against, that is in contact with, the skin tissue.

The negative pressure chamber 26 illustrated with dashed lines in Fig. 1A may essentially be a recess or cavity in or defined by the treatment head 100 or the first body part 11 into which skin tissue is intended to be sucked into by the negative pressure.

In some embodiments, a second body part 30 may be adapted for coupling removably with the first body part 11 at the first end and configured to be in fluid communication with a negative pressure source of the negative pressure treatment device. Furthermore, the second body part 30 may be adapted to function as a handle. The handle may be conveniently utilized for moving the treatment head during a treatment session.

In various embodiments, the second body part 30 may comprise at least one of the following: electronics, valves, electrical conductors 80, electrical circuits, data connections, low-pressure lines, etc., for operating the treatment head 100 and/or providing the negative pressure to the negative pressure chamber 26. The treatment head 100 may further comprise a sealing portion 22, preferably at least partly in the first body part 11, arranged to seal the coupling between the first 11 and the second body parts 30. The sealing portion 22 may comprise a sealing part 24, such as of rubber or other suitable material, for example, silicone. The sealing part 24 may be ring- or ellipse-shaped.

The first body part 11 may comprise a slot 25 for ensuring that the first body part 11 is attached in correct way, that is in right position, to a second body part 30, for instance. The second body part 30 may, thus, preferably comprise a counterpart, such as a protrusion or a peg, or an element fitting to slot 25. Of course, the slot 25 may also be comprised in the second body part 30 and the counterpart in the first body part 11.

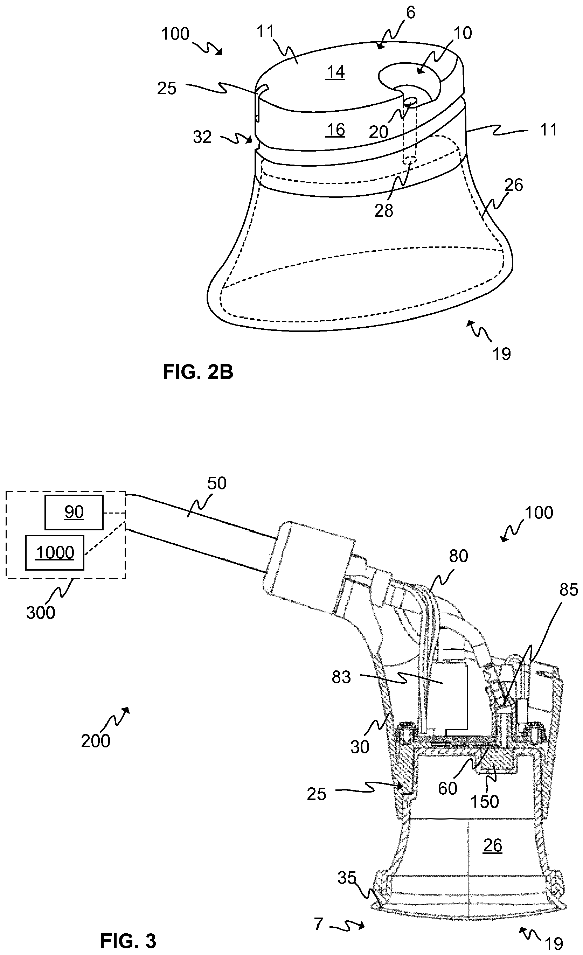

Figures 2A and 2B illustrate schematically treatment heads 100 according to some embodiments of the present invention. As shown in Figs. 2A and 2B, the sealing portion 22 may comprise a groove 32 on the surface of the first body part 11. The sealing part 24 may be arranged into the groove 32.

In various embodiments, the recess 10 may be arranged closer to the first end 6 than the sealing portion 22 such that the recess 10 is arranged to be in a sealed space between the first 11 and second 30 body parts such that the leakage of the air such as to bypass the second body part 30 through the first opening 12 is being minimized. In an embodiment, the sealing portion 22 may comprise a groove 32 in the first body part 11, wherein the groove 32 is arranged around the first body part 11 along the outer side surface 16. As illustrated in Fig. 1A, for instance, the sealing part 24 may be arranged into the groove, such as an O-ring of rubber or silicone.

In some embodiment, the groove 32 may be askew with respect to a plane being perpendicular to a longitudinal direction of the treatment head 100. The skewed groove 32 further strengthens the sealing portion 22 in order to prevent the sealing part 24 coming off when coupling the second body part 30 to the first body part 11. The skewed groove 32 provides advantage over straight groove 32 in that when arranging the second body part 30 to be in contact with the first body part 11, initially less amount of sealing part 24 becomes in contact with the second body part 30.

In various embodiments, the sealing portion 22 may, instead of being arranged to the first body part 11, be arranged to the second body part 30.

In various embodiments, a sensor 60 may be arranged and configured for monitoring a presence of the filter 150 in the recess 10, wherein the sensor 60 may be configured to provide an electrical signal representing the presence of the filter 150, for example, to a controlling unit 1000.

Figure 3 illustrates schematically a negative pressure treatment device 200 according to an embodiment of the present invention. The negative pressure treatment device 200 may comprise a treatment head 100 according to various embodiments presented hereinabove, such as comprising a first body part 11, the first body part 11 comprising an outer side surface 16 and an end surface 14. The first body part 11 may define a recess 10 for a filter 150 and a first opening 12 of the recess for arranging the filter 150 to the recess 10. The first opening 12 may be defined in part by the end surface 14 and in part by the outer side surface 16 for facilitating a removal of the filter 150 from the recess 10.

Furthermore, the negative pressure treatment device 200 amy comprise a main unit 300 comprising a controlling unit 1000 and a negative pressure source 90, such as a vacuum pump or a fan. The device 200 may comprise a low-pressure hose or line 50, such as being a flexible or rigid element, arranged for providing negative pressure produced by the negative pressure source 90 to the treatment head 100.

In some embodiments, the treatment head 100 may comprise a sealing element 35, such as being flexible, arranged at the suction opening 19 for sealing contact between the suction opening 19 and skin tissue.

In various embodiments, the main unit 300 may be connected to the treatment head 100 by the low-pressure hose or line 50, and electrical and, optionally, data communication connection means. In some embodiments, these may be integrated into one element comprising both the low-pressure hose or line 50 and the electrical and, optionally, data communication connection means.

According to various embodiments, the device 200, or specifically the main unit 300 thereof, may comprise a controlling unit 1000. There may be external unit(s) which may be connected to a communication interface of the controlling unit 1000. External unit(s) may comprise wireless connection or a connection by a wired manner. The communication interface may provide interface for communication with the external unit(s) such as user interfacing devices or the like. There may also be connection to an external system, such as a laptop or a handheld device. There may also be a connection to a database of the device 200 including information used in controlling the operation of the device 200.

The controlling unit 1000 may comprise one or more processors, one or more memories being volatile or non-volatile for storing portions of computer program code and any data values and possibly one or more user interface units. The mentioned elements may be communicatively coupled to each other with e.g. an internal bus. The processor may thus be arranged to access the memory and retrieve and store any information therefrom and thereto. For sake of clarity, the processor herein refers to any unit suitable for processing information and control the operation of the controlling unit 1000 among other tasks. The operations may also be implemented with a microcontroller solution with embedded software. Similarly, the memory is not limited to a certain type of memory only, but any memory type suitable for storing the described pieces of information may be applied in the context of the present invention.

The controlling unit 1000 may preferably be configured to be in connection with valve or valves in the main unit 300 and/or the treatment head 100. For example, there may be a valve 83 arranged at or close to the connection point 85 of the low-pressure hose or line 50 to the treatment head 100.

In various embodiments, the valve 83 may be arranged in various other locations, such as to the main unit, to the low-pressure hose 50, or anywhere in the treatment head 100 such as it may be utilized to control the negative pressure provided into the negative pressure chamber 26.

In some embodiments, the valve 83 may be a three-way valve 83 for providing either negative pressure from the negative pressure source 90 via the hose 50, or the ambient or surrounding pressure to be in fluid communication with the negative pressure chamber 26.

In various embodiment, the treatment head 100 may be attached to a low-pressure hose 50 for providing negative pressure from a negative pressure source 90 to the negative pressure chamber 26 in connection with the suction opening 19.

In some embodiments, the low-pressure hose or line 50 may be attached to the treatment head 100 for providing the negative pressure via the first 12 and the second 20 openings to the negative pressure chamber 26 in connection with the suction opening 19.

The specific examples provided in the description given above should not be construed as limiting the applicability and/or the interpretation of the appended claims. Lists and groups of examples provided in the description given above are not exhaustive unless otherwise explicitly stated.

## Claims

1. A treatment head (100) for a negative pressure treatment device (200), wherein the treatment head (100) comprises a first body part (11), the first body part (11) comprising an outer side surface (16) and an end surface (14), wherein the first body part (11) defines a recess (10) for a filter (150) and a first opening (12) of the recess for arranging the filter (150) to the recess (10), wherein the first opening (12) is defined in part by the end surface (14) and in part by the outer side surface (16) for facilitating a removal of the filter (150) from the recess (10).

2. The treatment head (100) according to claim 1, comprising a suction opening (19) at an opposite end (7) of the first body part (11) with respect to a first end (6) comprising the end surface (14).

3. The treatment head (100) according to claim 2, wherein the suction opening (19) is defined by the first body part (11).

4. The treatment head (100) according to any one of the preceding claims, comprising a second opening (20) defined by the first body part (11) in fluid communication with the first opening (12).

5. The treatment head (100) according to claim 4, wherein the filter (150) is arrangeable to the recess (10) between the first (12) and second (20) openings for filtering air flowing between said openings (12, 20).

6. The treatment head (100) according to claim 4 or 5, wherein the second opening (20) is in fluid communication with the suction opening (19) through a negative pressure chamber (26).

7. The treatment head (100) according to any one of claims 3-6, comprising a second body part (30) adapted for coupling removably with the first body part (11) at the first end (6) and configured to be in fluid communication with a negative pressure source (90) of the negative pressure treatment device (200).

8. The treatment head (100) according to claim 7, wherein the second body part (30) is adapted to function as a handle.

9. The treatment head (100) according to claim 7 or 8, comprising a sealing portion (22) arranged to seal the coupling between the first (11) and the second (30) body parts.

10. The treatment head (100) according to claim 9, wherein the recess (10) is arranged closer to the first end (6) than the sealing portion (22) such that the recess (10) is arranged to be in a sealed space between the first (11) and second (30) body parts.

11. The treatment head (100) according to claim 9 or 10, wherein the sealing portion (22) comprises a groove (32) in the first body part (11), wherein the groove (32) is arranged to extent around the first body part (11) along the outer side surface (16).

12. The treatment head (100) according to claim 10, wherein the groove (32) is askew with respect to a plane perpendicular to a longitudinal direction of the treatment head (100).

13. The treatment head (100) according to any one of the preceding claims, comprising a sensor (60) configured for monitoring a presence of the filter (150) in the recess (10), wherein the sensor (60) is configured to provide an electrical signal representing the presence of the filter (150).

14. A negative pressure treatment device (200) comprising a treatment head (100) according to any one of the preceding claims, wherein the negative pressure treatment device (200) further comprises a main unit (300) comprising a controlling unit (1000) and a negative pressure source (90), and a low-pressure hose or line (50) arranged for providing negative pressure produced by the negative pressure source (90) to the treatment head (100).

15. The negative pressure treatment device (200) according to claim 14, wherein the low-pressure hose or line (50) is attached to the treatment head (100) for providing the negative pressure via the first (12) and the second (20) openings to the negative pressure chamber (26) in connection with the suction opening (19).
